(19) Europäisches Patentamt / European Patent Office / Office européen des brevets

(11) **EP 1 870 705 B1**

(12) **FASCICULE DE BREVET EUROPEEN**

(45) Date de publication et mention
de la délivrance du brevet:
**17.08.2011 Bulletin 2011/33**

(51) Int Cl.:
*G01N 29/14* $^{(2006.01)}$     *G01N 29/44* $^{(2006.01)}$
*G01N 29/46* $^{(2006.01)}$     *G01N 33/34* $^{(2006.01)}$
*G01N 3/08* $^{(2006.01)}$

(21) Numéro de dépôt: **07290657.1**

(22) Date de dépôt: **23.05.2007**

(54) **Procédé et dispositif de mesure de la rigidité d'un échantillon de papier**

Verfahren und Vorrichtung zur Messung der Steifigkeit eines Papiermusters

Method and device for measuring the rigidity of a paper sample

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **31.05.2006 FR 0651993**

(43) Date de publication de la demande:
**26.12.2007 Bulletin 2007/52**

(73) Titulaire: **Georgia-Pacific France
92270 Bois-Colombes (FR)**

(72) Inventeur: **Leveugle, Denys
68240 Kaysersberg (FR)**

(74) Mandataire: **Cortier, Sophie et al
Georgia-Pacific France
Service Propriété Industrielle
60, avenue de l'Europe
92270 Bois-Colombes (FR)**

(56) Documents cités:
**FR-A1- 2 810 111**     **US-A- 3 683 681**
**US-A- 4 463 607**     **US-A- 4 596 152**

## Description

La présente invention concerne le domaine des produits cellulosiques fibreux, le papier notamment et plus particulièrement les papiers que l'on désigne couramment par l'expression papiers sanitaires et domestiques. Elle vise un procédé et un dispositif de mesure de la rigidité de ces produits.

La perception de la douceur d'un échantillon de ce type de produit que l'on tient dans la main dépend de plusieurs paramètres dont la rigidité, ou son opposée la souplesse, et la résilience.

Par ailleurs, on cherche à estimer dans certains cas l'effet d'un traitement sur la rigidité du produit final. Ainsi, par exemple il serait souhaitable de pouvoir suivre d'une manière simple l'évolution de la rigidité d'un papier en fonction du raffinage de la pâte.

On a déjà proposé d'analyser le son que le papier émet quand il est manipulé, froissé, frotté pour en caractériser certaines de ses propriétés.

Ainsi, le document US 3 683 681 décrit un dispositif agencé pour, en continu, froisser une bande de papier et en donner une mesure d'une caractéristique par un signal sonore audible qui dépend des ultrasons émis par le froissement.

Le document US 4 869 101 décrit un dispositif conçu pour, en continu également, frotter une bande de papier contre un film piézoélectrique dont les signaux qui en résultent peuvent être soumis à un analyseur de spectre de fréquences. Le spectre obtenu peut alors caractériser le produit. Une autre version de cette approche est divulguée dans le document FR 2 810 111.

Cependant ces documents ne fournissent pas vraiment de procédé de caractérisation quantitative et s'en tiennent à la description de moyens d'évaluation.

La demanderesse a donc recherché un procédé de mesure quantitative à la fois performant et objectif.

Le procédé selon l'invention de mesure de la rigidité (D) d'un papier ou autre produit cellulosique fibreux est caractérisé par le fait qu'on déchire un échantillon du papier, on enregistre le son produit pendant le déchirement, et on analyse le son enregistré de manière à mesurer un pourcentage (pc) de la présence de fréquences caractéristiques du déchirement, ce pourcentage étant un indicateur de la rigidité du papier.

L'invention met donc à la disposition du fabricant de papier une méthode simple mettant en oeuvre des moyens disponibles aisément. Plus précisément le procédé est caractérisé par le fait qu'il comporte les étapes suivantes :

- on déchire dans une première étape le papier avec une force prédéterminée ;
- on enregistre, dans cette étape, et on numérise les sons enregistrés à une fréquence d'échantillonnage et avec une résolution prédéterminée pour obtenir un enregistrement numérique d'une durée prédéterminée ;
- on analyse, dans une deuxième étape, l'enregistrement numérique dans les domaines temporel et fréquentiel et on mesure un pourcentage de la présence de fréquences caractéristiques du déchirement sur ladite durée prédéterminée pour en déduire un indice de rigidité.

De préférence, le papier est déchiré dans la direction de son sens marche.

Par ce procédé aux règles rigoureusement préétablies, on a pu établir au moins une relation linéaire de calcul de l'indice de rigidité en fonction du pourcentage de la présence desdites fréquences caractéristiques dans l'ensemble des spectres de fréquences obtenus sur la durée de l'enregistrement. De préférence pour cela :

- On effectue une transformation de Fourier (FFT) à partir de l'enregistrement numérique pour obtenir ledit ensemble des spectres codé en amplitudes selon une échelle de couleurs de façon à différencier les fréquences d'amplitudes au moins égales à une amplitude minimale prédéterminée et suffisamment pour pouvoir en effectuer la sélection.
- On limite ledit ensemble des spectres à une zone dite de test ne comportant que les fréquences supérieures à une fréquence minimale prédéterminée et on calcule dans cette zone de test la surface relative occupée par les fréquences d'amplitudes au moins égales à ladite amplitude minimale.
- On calcule l'indice de rigidité à partir de ladite surface relative.

Avantageusement, la zone de test comportant un nombre total de pixels prédéterminé, la mesure de la surface relative déterminant l'indice de douceur est le nombre de pixels couleur présents sur ladite surface, évaluable aisément grâce à une fonction histogramme.

L'invention concerne aussi un dispositif de mesure de la rigidité d'un échantillon de papier pour la mise en oeuvre du procédé ci-dessus, caractérisé par le fait qu'il comporte un déchiromètre, un micro, et, relié au micro, des moyens de calcul équipés d'une carte son et de modules d'enregistrement des sons et de traitement de signal.

On note que par ce procédé et grâce au système le mettant en oeuvre, on obtient une mesure objective de la rigidité du papier, parfaitement répétitive.

D'autres caractéristiques et avantages de la présente invention apparaîtront plus clairement à la lecture de la description ci-après, faite en référence au dessin annexé sur lequel :

- la figure 1 est un schéma par blocs fonctionnels du système permettant la mise en oeuvre du procédé de mesure de la rigidité selon l'invention,
- la figure 2 représente un organigramme des étapes effectuées pour la mise en oeuvre du procédé de mesure de la rigidité selon l'invention,
- la figure 3 représente un enregistrement temporel du son obtenu au moment du déchirement de papier,
- la figure 4 représente un ensemble de spectres de fréquences sur la durée de l'enregistrement temporel, codé selon une échelle de couleurs et
- la figure 5 représente le même ensemble de spectres limité à la zone de test.
- la figure 6 montre un équipement pour effectuer le déchirement d'un échantillon.

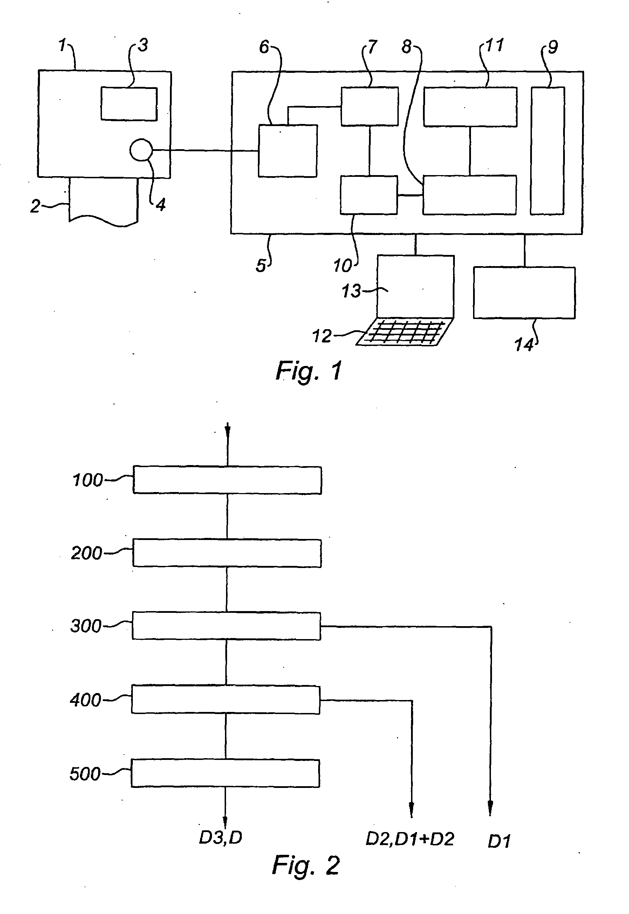

Le procédé proposé ici pour mesurer la rigidité d'un échantillon de papier consiste, en référence à la figure 1, à utiliser un système de mesure comportant un déchiromètre 1 équipé d'un micro 4 relié à des moyens de calcul, par exemple un ordinateur personnel ou PC (personal computer) 5. Le déchiromètre est un appareil connu en soi que l'on utilise par exemple pour mesurer des valeurs de résistance à la déchirure. Il est ici détourné de son utilisation habituelle.

Le déchiromètre 1 est par exemple de la marque Adamel-Lhomargy modèle ED20.

Pour le type de papier à mesurer, par exemple du papier hygiénique (PH) de taille 10 sur 12 centimètres, on leste le balancier 3 du déchiromètre d'un poids prédéterminé P égal à 350 grammes pour une échelle de mesure allant de zéro à 800 cN.

Il est classiquement prévu sur le déchiromètre un couteau pour réaliser une amorce de déchirure (non représenté).

Le micro 4, ici de la marque Shure SM58, est positionné de façon à pouvoir capter les sons ci-dessus dans de bonnes conditions.

Le déchiromètre est dépourvu de tout dispositif susceptible de générer des bruits parasites au cours de l'enregistrement des sons à capter. On a ici notamment enlevé son système de déblocage, mais ce n'est pas forcément nécessaire.

L'ordinateur personnel PC 5 est exploité ici sous le système d'exploitation Windows®. Il est équipé d'une carte son 6, ici une carte Terratec modèle Sixpack 5.1+, et de modules fonctionnels, composés essentiellement de logiciels, notamment un logiciel d'enregistrement 7, ici Nero Wave Editor de la marque Ahead Software, un logiciel standard d'analyse et de traitement de signal 8 tel que Spectrogram v8.8 distribué par Visualization Software, et un logiciel graphique 9 tel que Photoshop v7.0 d'Adobe.

Le logiciel 7 enregistre les données sonores issues de la carte son 6 sous forme numérique dans une première mémoire 10.

Le logiciel 8 traite les données sonores de la première mémoire 10 et mémorise ses données d'analyse et de traitement dans une seconde mémoire 11 comme il va être précisé ci-après.

Le PC 5 est relié à une interface homme-machine comportant un clavier 12 et un écran 13 capable de visualiser le contenu des mémoires 10 et 11, et accessoirement à une imprimante 14, du type imprimante universelle capable d'imprimer l'image présente sur l'écran 13, mais aussi de scanner les documents qu'elle imprime.

Le procédé comporte alors les étapes suivantes, en référence à la figure 2 :

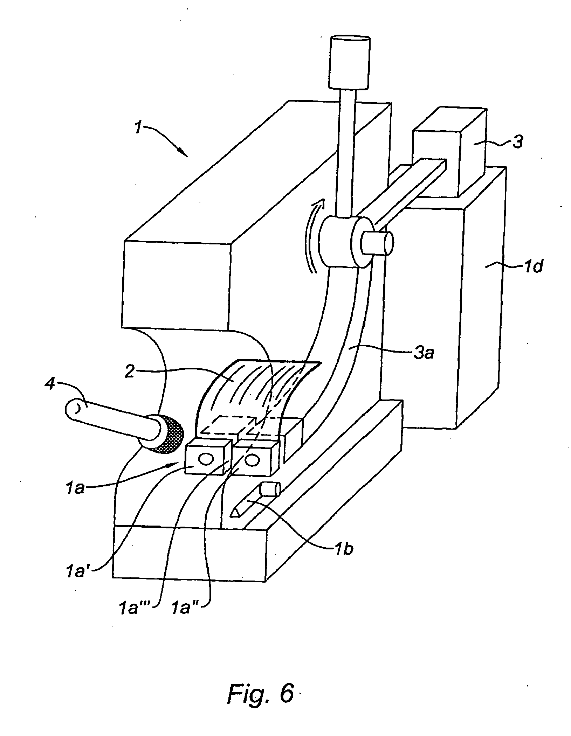

- Lors d'une première étape 100, on déchire le papier avec la force prédéterminée par le poids P. Pour cela, on place le papier 2 dans le déchiromètre 1 de façon à ce que la direction de fabrication du papier soit dans la direction de la déchirure. Selon la figure 6, l'échantillon 2 est placé dans la mâchoire 1a du déchiromètre 1. Cette mâchoire est formée de deux parties, l'une fixe 1a', l'autre mobile 1a", un espace libre 1a ter est ainsi ménagé entre les deux parties. Le couteau 1b est mis en mouvement de manière à passer dans l'espace libre 1 ter entre les deux mâchoires et effectuer une amorce de déchirure dans la direction de la déchirure correspondant à environ 1/6 de la longueur de l'échantillon dans la direction de fabrication. Le balancier 3 est dans une position d'arrêt, une cale 1d bloquant la masse du balancier. On retire la cale 1d du balancier en le maintenant en position de départ au moyen du bras de levier 3a et on lance l'enregistrement du son. On relâche le bras de levier 3a du balancier 3 prestement, ce qui ne génère aucun bruit parasite. Le balancier 3 ainsi libéré, pivote et met en mouvement la mâchoire mobile 1a" entraînant la déchirure complète de l'échantillon.
- Pendant cette étape 100, le déchirement émet des sons captés par le micro 4 et enregistrés grâce à la carte son 6 et au logiciel 7 à une fréquence d'échantillonnage fe assez élevée, ici de 44100 Hertz avec une résolution r de 16 bits. Le logiciel 7 enregistre les sons sous forme numérique dans la mémoire 10. L'enregistrement numérique mémorisé est d'une durée suffisante T pour couvrir toute la durée du son émis, grâce au logiciel 7.
- Lors d'une deuxième étape 200 suivante, on analyse l'enregistrement numérique de la mémoire 10 dans les domaines temporel et fréquentiel de la façon suivante :

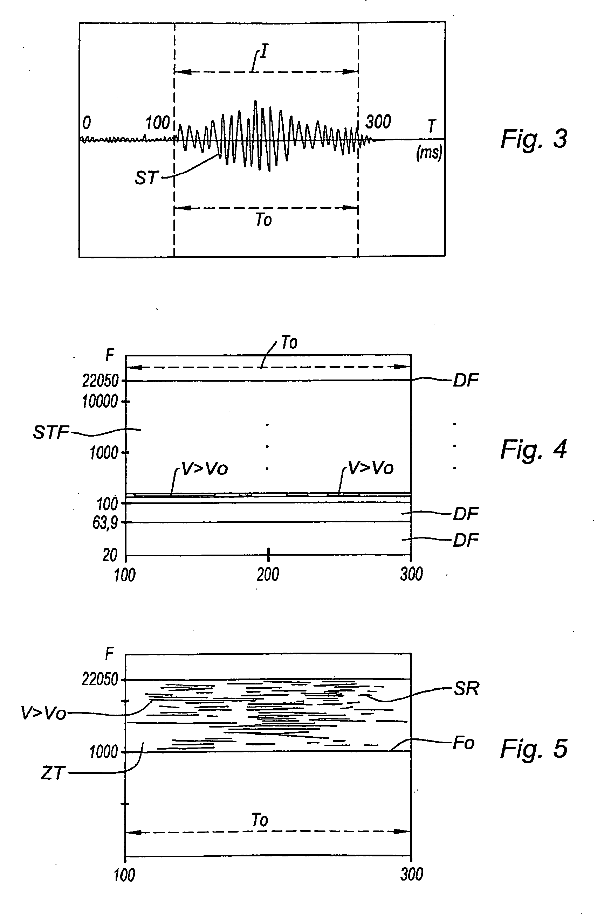

1) Dans l'enregistrement numérique, on sélectionne, grâce au clavier 12 et par le logiciel 7, un intervalle I de temps de durée prédéterminée ici 200 millisecondes compris entre $T_0$ + 100ms et $T_0$ + 300ms , $T_0$ étant le début du spectre sonore. On obtient ainsi un enregistrement numérique réduit ou « spectre temporel » ST tel que montré en figure 3. L'intervalle de temps I de durée T est transmis au logiciel de traitement de signal 8 sous un format compatible aux deux logiciels 7 et 8, par exemple WAV (Windows Audio Video) qui est un format de fichiers son de Windows.

2) Le logiciel 8 effectue des transformations de Fourier rapides (FFT) sur le spectre ST, ici de taille 1024 points, donc avec une résolution DT, ici de 0,195 milliseconde, pour obtenir, en référence à la figure 4, un ensemble STF de spectres « temps-fréquence » en échelle temporelle linéaire et fréquentielle logarithmique, comportant ici des fréquences F de 20 à 22050 Hertz avec une résolution DF égale à 43,1 Hertz. Les amplitudes sont codées en couleur selon une échelle de couleurs répartie de façon à bien différencier les fréquences F d'amplitudes V supérieures à une amplitude minimale prédéterminée Vo (V>Vo sur la figure), et ici correspondant à un volume sonore de 20 dB. Toutes ces valeurs sont indicatives et on pourrait définir d'autres réglages, dans la mesure où on en conserve la cohérence.

Les amplitudes V doivent être suffisamment différenciées par les couleurs choisies pour pouvoir être ultérieurement sélectionnées, par exemple coloriées en couleurs sombres. Sur la figure 4, les couleurs ne sont pas représentées, mais la sélection potentielle est rendue effective par des hachures noires horizontales. Une simple comparaison effectuée par logiciel peut permettre d'obtenir automatiquement cette sélection.

L'image de l'ensemble STF fourni par le logiciel 8 est mémorisée en mémoire 11 et peut alors être transmise au logiciel graphique 9 sous forme d'une nouvelle image montrée en figure 5, de taille identique à la définition de l'écran du PC, ici 1024x1280 pixels couleur. Cette transmission peut par exemple être effectuée en opérant une impression d'écran (touche « impr écran syst » du clavier 12) sur l'imprimante 14, le document ainsi imprimé étant ensuite scanné sur la même imprimante 14 et l'image obtenue ramenée par le logiciel 9 à une image 1024x1280.

- Lors d'une étape 300 suivante, on mesure le pourcentage de présence de fréquences Fc ,dites caractéristiques, sur la durée To, en utilisant le logiciel 9, lequel effectue leur sélection par le moyen évoqué ci-dessus. On pourrait opérer manuellement sur le document imprimé en utilisant un aréomètre. Ces fréquences caractéristiques Fc sont les fréquences F présentes dans l'intervalle I de durée To, d'amplitudes V supérieures à Vo et supérieures à une fréquence minimum Fo prédéterminée. Elles sont considérées comme suffisamment caractéristiques des sons émis lors du déchirement du papier 2. Plus précisément :

1) On limite l'ensemble STF des spectres temps-fréquence, en référence à la figure 5, à une zone ZT dite de test comportant les fréquences F supérieures à la fréquence minimale Fo prédéterminée. Dans l'exemple choisi Fo = 1000 Hertz. Puis on calcule dans cette zone ZT la surface relative SR des zones sombres occupée par les fréquences F d'amplitudes V supérieures à Vo. Pour cela, la zone ZT comporte un nombre total de pixels couleur NTP prédéterminé et toujours identique (1024x1280). En fait, la mesure de la surface relative SR occupée par les fréquences Fc est alors parfaitement représentée par le nombre de pixels couleur N1 présents sur cette surface SR. Et le nombre N1 est lui-même parfaitement représentatif du pourcentage pc de présence des fréquences F cherché puisque :

$$pc = 100 \times N1 / NTP \qquad\qquad (20)$$

Il est pratique d'utiliser directement le nombre N1 puisqu'il peut être évalué simplement en faisant effectuer un histogramme des surfaces coloriées en sombre sur la zone ZT par le logiciel 9.

Sans être lié par la théorie, il ressort de cette méthode qu'il existerait une relation étroite entre la cohésion, les liaisons inter-fibres et le son du déchirement. Les liaisons inter-fibres peuvent être provoquées par un raffinage plus ou moins fort de la suspension de fibres ou un pressage en partie humide. L'analyse du son du déchirement présente un intérêt certain pour les papiers tissues dits « stratifiés » qui peuvent comporter une strate intérieure de fibres plus résistantes, plus longues et/ou plus raffinées et une strate extérieure de fibres plus douces, plus courtes et peu liées.

Comme les mesures effectuées selon le procédé ci-dessus ne dépendent que d'évaluations physiques et non subjectives, elles sont fidèles aux erreurs de mesure physiques près, ce qui était bien le but recherché.

**EP 1 870 705 B1**

**Revendications**

1. Procédé de mesure de la rigidité (D) d'un papier (2) ou autre produit cellulosique fibreux **caractérisé par le fait qu'**on déchire un échantillon du papier, on enregistre le son produit pendant le déchirement, et on analyse le son enregistré de manière à mesurer un pourcentage (pc) de la présence de fréquences caractéristiques du déchirement, ce pourcentage étant un indicateur de la rigidité du papier.

2. Procédé de mesure de la rigidité (D) d'un échantillon de papier (2) ou autre produit cellulosique fibreux selon la revendication 1, **caractérisé par le fait qu'**il comporte les étapes suivantes :

   - On déchire le papier (2) dans une première étape (100) avec une force prédéterminée (P) ;
   - on enregistre (7) durant cette étape (100) et on numérise (7) les sons enregistrés à une fréquence d'échantillonnage (fe) et avec une résolution (r) prédéterminées pour obtenir un enregistrement numérique (10, ST) d'une durée (T) prédéterminée ;
   - on analyse, dans une deuxième étape (200), l'enregistrement numérique (ST) dans les domaines temporel et fréquentiel (STF) et on mesure un pourcentage (pc) de la présence des fréquences caractéristiques (Fc) du déchirement sur ladite durée prédéterminée (T) pour en déduire un indice de rigidité (D).

3. Procédé selon la revendication 1 ou 2, dans lequel on déchire le papier (2) dans son sens marche.

4. Procédé selon l'une des revendications 2 et 3, dans lequel on utilise au moins une relation linéaire (20, 21) de calcul de l'indice de rigidité (D) en fonction du pourcentage (pc) de la présence desdites fréquences (Fc) dans l'ensemble (STF) des spectres de fréquences (STF) obtenus sur la durée (T) de l'enregistrement (ST).

5. Procédé selon l'une des revendications 2 à 4, dans lequel, pour analyser ledit enregistrement numérique (ST) et mesurer ledit pourcentage (pc) :

   - On effectue une transformation de Fourier à partir de l'enregistrement numérique (ST) pour obtenir ledit ensemble (STF) codé en amplitudes selon une échelle de couleurs de façon à différencier les fréquences (Fc) d'amplitudes (V) au moins égales à une amplitude minimale (Vo) prédéterminée et suffisamment pour pouvoir en effectuer la sélection (V>Vo).
   - On limite ledit ensemble des spectres (STF) à une zone (ZT) ne comportant que les fréquences (Fc) supérieures à une fréquence minimale (Fo) prédéterminée et on calcule dans cette zone de test la surface relative (N1) occupée par les fréquences (Fc) d'amplitudes (V) au moins égales à ladite amplitude minimale (Vo).
   - On calcule l'indice de rigidité (D) à partir de ladite surface relative (N1).

6. Procédé selon la revendication 5, dans lequel les fréquences caractéristiques (Fc) sont celles obtenues sur la durée (T) de l'enregistrement numérique (ST), supérieures à la fréquence minimale (Fo) et d'amplitude (V) au moins égale à l'amplitude minimale (Vo).

7. Procédé selon l'une des revendications 5 et 6, dans lequel, la zone (ZT) comportant un nombre total (NTP) de pixels couleur prédéterminé, la mesure de la surface relative (SR) déterminant l'indice de rigidité (D) est le nombre de pixels (N1) présents sur ladite surface (SR).

8. Procédé selon l'une des revendications 5 à 7, dans lequel le calcul de l'indice de rigidité (D) est une fonction linéaire (21) de la mesure de la surface relative (N1).

9. Dispositif de mesure de la rigidité (D) d'un échantillon de papier (2) ou autre produit cellulosique fibreux pour la mise en oeuvre du procédé selon l'une des revendications 1 à 8, **caractérisé par le fait qu'**il comporte un déchiromètre agencé pour déchirer un échantillon de papier, (1), un micro (4) agencé pour capter le son produit pendant le déchirement et, reliés au micro (4), des moyens de traitement du signal (5) équipés d'une carte son (6) et de modules (7-11) d'enregistrement des sons et de traitement de signal, agencés pour mesurer un pourcentage (pc) de la présence de fréquences caractéristiques du déchirement, ce pourcentage étant un indicateur de la rigidite du papier.

## Claims

1. Method of measuring the rigidity (D) of a paper (2) or other cellulosic fibrous product **characterized in that** a sample of the paper is torn, the sound generated during the tearing is recorded and the recorded sound is analysed so as to measure a percentage (pc) of the presence of frequencies characteristic of the tearing, this percentage being an indicator of the rigidity of the paper.

2. Method of measuring the rigidity (D) of a sample of paper (2) or other cellulosic fibrous product according to Claim 1, **characterized in that** it comprises the following steps:

   - the paper (2) is torn in a first step (100) with a predetermined force (P);
   - a recording (7) is made during this step (100) and a digitization (7) is performed on the recorded sounds at a predetermined sampling frequency (fe) and with a predetermined resolution (r) to obtain a digital recording (10, ST) of a predetermined duration (T);
   - in a second step (200), the digital recording (ST) is analysed in the temporal and frequency domains (STF) and a percentage (pc) of the presence of the frequencies (Fc) characteristic of the tearing is measured over the said predetermined duration (T) in order to deduce a rigidity index (D) therefrom.

3. Method according to Claim 1 or 2, in which the paper (2) is torn in its direction of travel.

4. Method according to one of Claims 2 and 3, in which use is made of at least one linear computational relation (20, 21) of the rigidity index (D) as a function of the percentage (pc) of the presence of the said frequencies (Fc) in the set (STF) of frequency spectra (STF) obtained over the duration (T) of the recording (ST).

5. Method according to one of Claims 2 to 4, in which, to analyse the said digital recording (ST) and measure the said percentage (pc):

   - a Fourier transform is carried out based on the digital recording (ST) to obtain the said set (STF) encoded in amplitudes according to a colour scale so as to differentiate the frequencies (Fc) of amplitudes (V) at least equal to a predetermined minimal amplitude (Vo) and sufficiently to be able to make the selection (V>Vo) thereof.
   - the said set of spectra (STF) is limited to a zone (ZT) comprising only the frequencies (Fc) greater than a predetermined minimal frequency (Fo) and, in this test zone, the relative surface area (N1) occupied by the frequencies (Fc) of amplitudes (V) at least equal to the said minimal amplitude (Vo) is computed.
   - the rigidity index (D) is computed based on the said relative surface area (N1).

6. Method according to Claim 5, in which the characteristic frequencies (Fc) are those obtained over the duration (T) of the digital recording (ST), greater than the minimal frequency (Fo) and with an amplitude (V) at least equal to the minimal amplitude (Vo).

7. Method according to one of Claims 5 and 6, in which the zone (ZT) comprising a predetermined total number (NTP) of colour pixels, the measurement of the relative surface area (SR) determining the rigidity index (D), is the number of pixels (N1) present on the said surface area (SR).

8. Method according to one of Claims 5 to 7, in which the computation of the rigidity index (D) is a linear function (21) of the measurement of the relative surface area (N1).

9. Device for measuring the rigidity (D) of a sample of paper (2) or other cellulosic fibrous product for the application of the method according to one of Claims 1 to 8, **characterized in that** it comprises a tear tester (1) arranged to tear a sample of paper, a microphone (4) arranged to sense the sound generated during the tearing and, connected to the microphone (4), signal processing means (5) fitted with a sound card (6) and sound recording and signal processing modules (7-11), which are arranged to measure a percentage (pc) of the presence of frequencies characteristic of the tearing, this percentage being an indicator of the rigidity of the paper.

## Patentansprüche

1. Verfahren zum Messen der Steifigkeit (D) eines Papiers (2) oder eines anderen faserartigen Zelluloseprodukts, **dadurch gekennzeichnet, dass** eine Probe des Papiers eingerissen wird, der während des Einreißens erzeugte Ton aufgezeichnet wird und der aufgezeichnete Ton analysiert wird, um einen Prozentsatz (pc) des Vorhandenseins von charakteristischen Einreißfrequenzen zu messen, wobei dieser Prozentsatz ein Indikator für die Steifigkeit des Papiers ist.

2. Verfahren zum Messen der Steifigkeit (D) einer Probe eines Papiers (2) oder eines anderen faserartigen Zelluloseprodukts nach Anspruch 1, **dadurch gekennzeichnet, dass** es die folgenden Schritte umfasst:

   - in einem ersten Schritt Einreißen des Papiers (2) (100) mit einer vorgegebenen Kraft (P);
   - Aufzeichnen (7) während dieses Schrittes (100) und Digitalisieren (7) der aufgezeichneten Töne mit einer vorgegebenen Abtastfrequenz (fe) und mit einer vorgegebenen Auflösung (r), um eine digitale Aufzeichnung (10, ST) mit einer vorgegebenen Dauer (T) zu erhalten;
   - in einem zweiten Schritt (200) Analysieren der digitalen Aufzeichnung (ST) im Zeitbereich und im Frequenzbereich (STF) und Messen eines Prozentsatzes (pc) des Vorhandenseins von charakteristischen Frequenzen (Fc) des Einreißens während der vorgegebenen Dauer (T), um daraus einen Steifigkeitsindex (D) abzuleiten.

3. Verfahren nach Anspruch 1 oder 2, wobei das Papier (2) in seiner Bewegungsrichtung eingerissen wird.

4. Verfahren nach einem der Ansprüche 2 und 3, wobei wenigstens eine lineare Beziehung (20, 21) zum Berechnen des Steifigkeitsindexes (D) als Funktion des Prozentsatzes (pc) des Vorhandenseins der Frequenzen (Fc) in der Gesamtheit (STF) von Frequenzspektren (STF), die während der Dauer (T) der Aufzeichnung (ST) erhalten werden, verwendet wird.

5. Verfahren nach einem der Ansprüche 2 bis 4, wobei zum Analysieren der digitalen Aufzeichnung (ST) und zum Messen des Prozentsatzes (pc):

   - eine Fourier-Transformation anhand der digitalen Aufzeichnung (ST) ausgeführt wird, um die amplitudencodierte Gesamtheit (STF) auf einer Farbskala zu erhalten, um die Frequenzen (Fc) abzuleiten, deren Amplituden (V) wenigstens gleich einer vorgegebenen minimalen Amplitude (Vo) sind, die genügend groß ist, um damit die Auswahl (V > Vo) vorzunehmen;
   - die Gesamtheit der Spektren (STF) auf eine Zone (ZT) eingeschränkt wird, die nur Frequenzen (Fc) enthält, die größer als eine vorgegebene minimale Frequenz (Fo) sind, und in dieser Testzone die relative Fläche (N1) berechnet wird, die von den Frequenzen (Fc) mit Amplituden (V), die wenigstens gleich der minimalen Amplitude (Vo) sind, eingenommen wird,
   - der Steifigkeitsindex (D) anhand dieser relativen Fläche (N1) berechnet wird.

6. Verfahren nach Anspruch 5, wobei die charakteristischen Frequenzen (Fc) jene sind, die während der Dauer (T) der digitalen Aufzeichnung (ST) erhalten werden und größer sind als die minimale Frequenz (Fo) mit einer Amplitude (V), die wenigstens gleich der minimalen Amplitude (Vo) ist.

7. Verfahren nach einem der Ansprüche 5 und 6, wobei dann, wenn die Zone (ZT) eine Gesamtzahl (NTP) von Pixeln mit vorgegebener Farbe enthält, die Messung der relativen Fläche (SR), die den Steifigkeitsindex (D) bestimmt, gleich der Anzahl von Pixeln (N1) ist, die in der Fläche (SR) vorhanden sind.

8. Verfahren nach einem der Ansprüche 5 bis 7, wobei das Berechnen des Steifigkeitsindexes (D) eine lineare Funktion (21) des Messwerts der relativen Fläche (N1) ist.

9. Vorrichtung zum Messen der Steifigkeit (D) einer Probe eines Papiers (2) oder eines anderen faserartigen Zelluloseprodukts, um das Verfahren nach einem der Ansprüche 1 bis 8 auszuführen, **dadurch gekennzeichnet, dass** es eine Einreißmesseinrichtung (1), die dazu ausgelegt ist, eine Papierprobe einzureißen, ein Mikrophon (4), das dazu ausgelegt ist, den während des Einreißens erzeugten Ton einzufangen, und mit dem Mikrophon (4) verbundene Signalverarbeitungsmittel (5), die mit einer Audio-Karte (6) und mit Modulen (7-11) für die Aufzeichnung der Töne und für die Signalverarbeitung ausgerüstet sind, die dazu ausgelegt sind, einen Prozentsatz (pc) des Vorhandenseins von charakteristischen Einreißfrequenzen zu messen, umfasst, wobei dieser Prozentsatz ein Indikator für die Stei-figkeit des Papiers ist.

Fig. 1

D3,D          D2,D1+D2      D1

Fig. 2

Fig. 3

Fig. 4

Fig. 5

*Fig. 6*

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3683681 A
- US 4869101 A

- FR 2810111